# EUROPEAN PATENT SPECIFICATION

(11) **EP 0 746 233 B1**
(45) Date of publication and mention of the grant of the patent: **31.10.2001**
(21) Application number: 94916498.2
(22) Date of filing: 22.02.1994
(51) Int. Cl.: A61B 8/12, A61B 18/24

(54) **CATHETER USING OPTICAL FIBERS TO TRANSMIT LASER AND ULTRASONIC ENERGY**
KATHETER MIT OPTISCHEN FASERN ZUM ÜBERTRAGEN VON LASER- UND ULTRASCHALLENERGIE
CATHETER UTILISANT DES FIBRES OPTIQUES POUR TRANSMETTRE L'ENERGIE LASER ET ULTRASONORE

(43) Date of publication of application: 11.12.1996
(73) Proprietor: Intraluminal Therapeutics, Inc., Carlsbad, CA 92009 (US)
(72) Inventor: WINSTON, Thomas, R., Leawood, KS 66211 (US); NEET, John, M., Lawrence, KANSAS 66049 (US)
(74) Representative: Quinterno, Giuseppe
(86) International application number: US9401987
(87) International publication number: WO9522283

(56) References cited:
- WO-A-93/07806
- DE-A- 4 322 955
- US-A- 4 887 605
- US-A- 5 010 886
- US-A- 5 152 291

## Description

### Field of the Invention

This invention relates in general to medical laser treatment of atherosclerotic plaque and other conditions. The invention deals more particularly with an apparatus that makes use of a catheter equipped with optical fibers, each of which transmits both ultrasonic imaging signals and laser energy for treatment of the medical condition.

### Background of the Invention

Lasers have been used in a variety of medical applications, including the treatment of atherosclerotic plaque which builds up on the walls of arteries and can create cardiovascular problems by restricting arterial flow. In order to properly apply the laser energy, it is necessary to obtain an image of the area that is to undergo treatment. For example, U.S. Patent No. 4,576,177 to Webster discloses a catheter which includes optical fibers for transmitting laser energy and an ultrasonic transducer at the tip end of the catheter for obtaining ultrasonic images. Electrical wires extend through the catheter to provide power for operating the ultrasonic transducer-and to transmit image information sensed by the transducer.

International application WO-A-9216140 filed on March 18, 1992 in the name of Thomas R. Winston for "Catheter for Laser Treatment of Atherosclerotic Plaque and other Abnormalities" discloses a number of different catheter constructions that improve upon the catheter shown in the Webster patent. International application WO-A-9314689 filed on January 14, 1993 in the names of Thomas R. Winston and John M. Neet for "Medical Catheter Using Ultrasound Mapping With External Transducers" discloses yet another improvement in which the ultrasonic transducer is external to the catheter.

In the arrangement shown in the latter application, separate optical fibers are used to transmit the ultrasonic signals and the laser energy. While this is entirely satisfactory in some applications, there are other applications in which the need to provide separate fibers for the ultrasound and laser energy is a significant disadvantage.

U.S. patent No. 4,587,972 to Morantte discloses a device for imaging and removing obstructions from blood vessels and the like, which device includes a flexible tubular housing in which a fiber optic bundle adapted to conduct laser beams to an obstruction in the vessel for the destruction thereof, and an ultrasound portion adapted to generate ultrasonic waves processable to create an image of the obstruction, are provided. An electrode portion connected to an external source of energy extends longitudinally within the housing to conduct an electrical charge therein to (from) a piezoelectric portion provided along the device and adapted to emit (receive) the ultrasonic waves.

### Summary of the Invention

The present invention is directed to an improved medical catheter in which one or more optical fibers are extended through a catheter, and each fiber transmits both ultrasonic signals used for ultrasonic imaging and laser energy used for treating the medical problem (arterial plaque build up, for example). The catheter may have a single fiber, multiple fibers arranged in a bundle, or multiple fibers arranged in multiple bundles. In all cases, each fiber is connected both to an ultrasonic transducer and to a laser so that it transmits the energy used for the ultrasonic imaging as well as the energy used for laser treatment.

Different arrangements may be provided to allow the same fiber to transmit both ultrasound and laser energy. In one embodiment which does not fall under the definition of the present invention, a Y-shaped fiber coupler having connects to the optical fiber which extends through the catheter. The ultrasonic transducer can be connected to one of the branches and the laser can be connected to the other branch. A modified way of using this same concept provides multiple fibers extending through the catheter, with each fiber having two branches for accommodating both ultrasound and laser energy.

According to the invention, the ultrasonic transducer is connected to the optical fiber and another smaller fiber passes through the transducer and connects with the main fiber. The laser energy can be applied to the smaller fiber and is transmitted from it to the main fiber and ultimately to the area that is undergoing treatment.

In a further embodiment which is also not covered by the claim, the ultrasonic transducer is connected with an optical filament that is smaller than the main fiber and which connects with the main fiber by means of a suitable coupler. A plurality of additional small filaments are arranged in a circle around the filament which transmits ultrasound, and these additional filaments are likewise coupled with the main optical fiber. By means of this arrangement, both the ultrasound and laser energy can be applied to the same main fiber, the ultrasound making use of the central filament and the laser energy being applied to the other filaments.

### Description of the Drawings

In the accompanying drawings which form a part of the specification and are to be read in conjunction therewith and in which like reference numerals are used to indicate like parts in the various views:
Fig. 1 is a diagrammatic side elevational view of a catheter, with the break lines indicating continuous length and the catheter shown inserted with its tip end in position to effect treatment of an arterial plaque deposit;
Fig. 2 is a diagrammatic side elevational view of a catheter which is similar to that shown in Fig. 1 but which includes a plurality of different optical fibers each of which transmits both ultrasound and laser energy;
Fig. 3 is a fragmentary end elevational view taken generally along line 3-3 of Fig. 2 in the direction of the arrows;
Fig. 4 is a fragmentary perspective view of a catheter constructed according to the invention; and
Fig. 5 is a fragmentary perspective view of a catheter constructed according to still another modified embodiment.

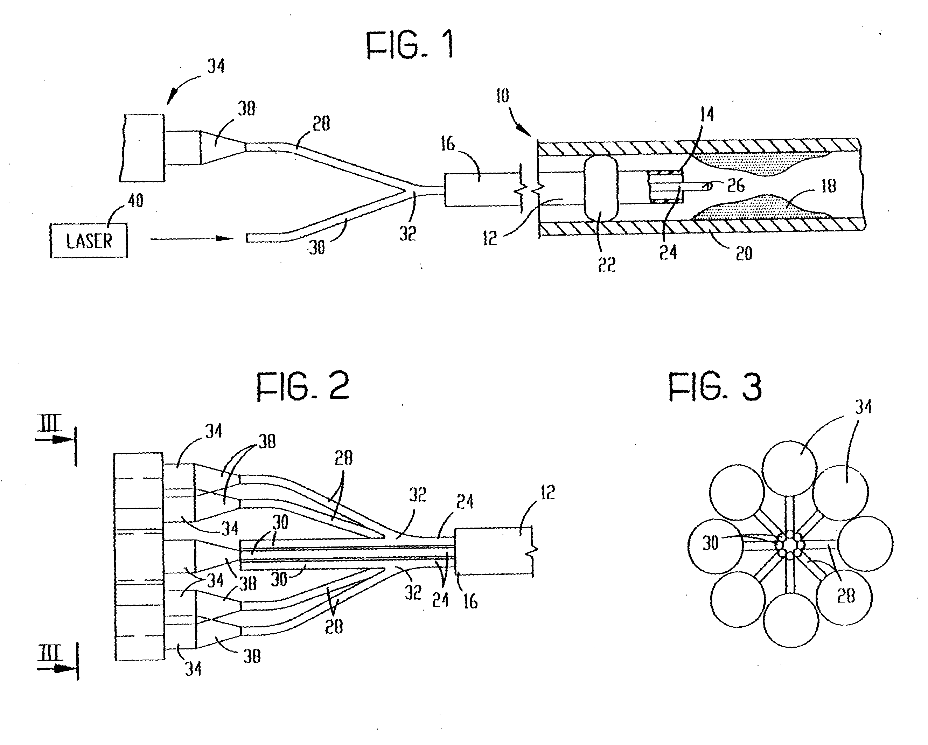

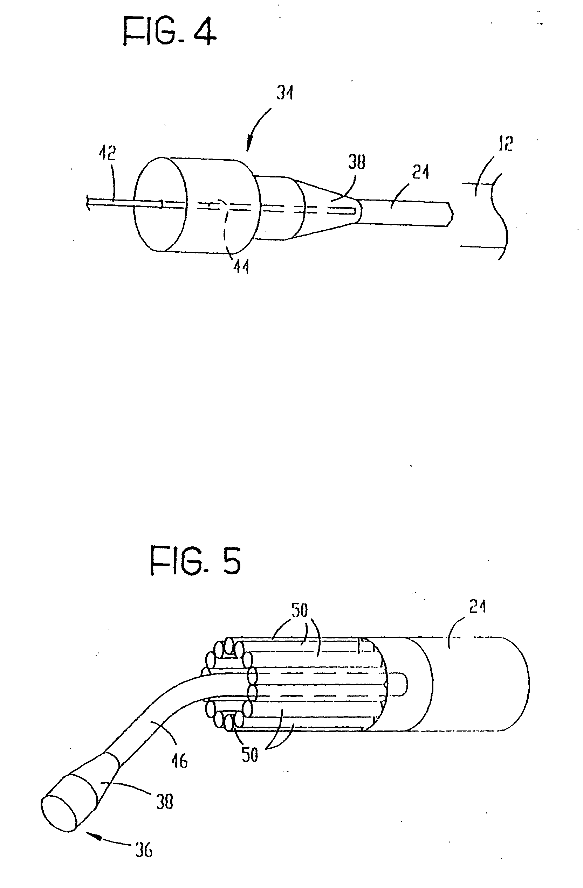

Although the embodiments corresponding to figures 1 to 3 and 5 are not covered by the claim, they appear to be useful for understanding the principle of the present invention as disclosed in relation with figure 4.

### Detailed Description of the Invention

Referring now to the drawings in more detail and initially to Fig. 1, a catheter is generally identified by numeral 10. The catheter 10 includes an elongated flexible catheter tube 12 which is hollow and preferably circular in crosssection. The catheter tube 12 is constructed in a suitable manner to be inserted into the body with one end 14 situated adjacent to the area that is to be treated and the other end 16 remaining external of the body. By way of example, Fig. 1 depicts use of the catheter 10 in the treatment of atherosclerotic plaque 18 which has built up on the wall of an artery 20. In this type of application, the distal end 14 of the catheter tube 12 is inserted into the artery 20 to the location of the plaque deposit 18. An annular seal 22 may be provided near the distal end 14 to effect a seal between the outside of the catheter tube 12 and the arterial wall.

An optical fiber 24 extends through the catheter tube 12 and terminates in a tip 26 which may project out through the distal end 14 of the catheter tube. The opposite or input end of the optical fiber 24 suitably connects with a Y-shaped fiber coupler having two branches 28 and 30 that merge at a junction 32. The fiber coupler is a commercially available article, and it may be fused or otherwise suitably connected end to end with the main optical fiber 24. The fiber 24 and the fiber coupler may be quartz or silicon fiber coated with an external reflective material.

A conventional ultrasonic transducer 34 is located outside of the catheter tube 12 and is excited electrically in the usual manner to transmit ultrasonic signals through a piezo-electrode. The transducer 34 connects with the free end of branch 28 through a tapered transition element 38. The ultrasonic signals which are transmitted to the transition element 38 by the transducer are transmitted along the branch 28 to the fiber 24 and are directed to the area that is to undergo treatment through the fiber tip 26. Pulse echoes which are reflections of the transmitted signals are received by the tip 26 and are transmitted along the fiber 24 and the branch 28 back to the transducer. The transducer transforms the received pulse echoes into electrical signals which provide data to form an image of the area that is to undergo treatment. In this fashion, the ultrasonic system provides images as to the configuration, location and character of the tissue in the area of the plaque 18. Preferably, the transducer is a piezo-electric ceramic crystal unit.

The tapered transition element 38 may be a solid frustoconical member constructed of a substance suitable to conduct ultrasonic signals. As an alternative, the transition element 38 may take the form of a hollow frusto-conical member that is filled with a fluid capable of conducting ultrasonic signals. The large end of the transition element 38 connects with the piezo-electrode 36, and the small end connects with the free end of the branch 28.

Laser energy is applied to the free end of the other branch 30 by a medical laser 40. The laser energy is applied to the branch 30 and is transmitted along the branch and the fiber 24 to the tip 26 which directs the laser energy toward the plaque 18 in order to remove the plaque 18 or at least reduce the plaque build up.

Figs. 2 and 3 depict an alternative embodiment in which the catheter tube 12 contains a plurality of the optical fibers 24. Each of the fibers 24 is fused or otherwise connected at its proximal end with a fiber coupler having a pair of branches 28 and 30. The branches 28 connect with respective transducers 34 via transition elements 38. Consequently, each of the fibers 24 receives ultrasonic signals from the corresponding transducer 34 and transmits reflected pulse echoes back to the corresponding transducer 34. In this fashion, the plural transducers 34 provide accurate ultrasonic imaging information of the area that is to undergo treatment.

The fibers 24 may be arranged in a circular pattern, and the branches 30 may be arranged in line with the respective fibers 24 such that the branches 30 are essentially in line extensions of the fibers. The extensions 30 are thus arranged in a circular pattern, as best shown in Fig. 3. The laser 40 may be used to apply laser energy to the free ends of the extensions 30, and the extensions and fibers 24 transmit the laser energy through the catheter tube 12 to the area undergoing treatment. Because of the circular arrangement of the fibers 24, the transducers 34 may be arranged in a circular pattern, as shown in Fig. 3.

Fig. 4 depicts an embodiment of the invention in which the transition element 38 is fused or otherwise connecred directly to the proximal end of the fiber 24. The transducer 34 applies ultrasonic signals to the fiber 24 through the transition element 38 in the manner previously described, and the pulse echoes are transmitted back to the transducer in the same fashion described previously as well.

The laser 40 applies laser energy to a small optical fiber or filament 42. The filament 42 may take the form of an optical fiber having a construction similar to that of the fiber 24 but a diameter considerably less than that of the fiber 24. The filament 42 extends through a passage 44 which is formed through the transducer 34 and transition element 38. The end of the filament 42 butts against the input end of the fiber 24 and is fused or otherwise suitably connected with the optical fiber. The laser energy which is applied to the filament 42 is thus applied to the input end of the optical fiber 24 and is transmitted along the optical fiber and applied to treat the medical problem that is undergoing treatment.

Fig. 5 depicts an alternative embodiment in which the transition element 38 connects with one end of an optical fiber or filament 46. The piezo-electrode of the transducer 34 is connected to the transition element 38 as previously described. The opposite end of the filament 46 extends to and connects with the proximal end of the optical fiber 24. The filament 46 may be constructed in a manner similar to the fiber 24, but filament 46 is considerably smaller in diameter than the fiber 24. The end of the fiber 46 is fused or otherwise suitably connected with the end of the fiber 24.

The transducer 34 applies ultrasonic signals to filament 46 which in turn applies the signals to the fiber 24. The pulse echoes are transmitted back to the transducer to provide ultrasonic imaging data of the area that is to undergo treatment.

A plurality of additional filaments 50 connect with the proximal end of fiber 24 (as by fusing, for example) and may be arranged in a circular pattern around the filament 46. The filaments 50 may be larger or smaller than the central filament 46, and they may be constructed in a manner similar to the fiber 24. However, the filaments 50 are preferably somewhat smaller in diameter than the central filament 46. The laser 40 operates to apply laser energy to the filaments 50, and the laser energy is transmitted along the filaments 50 to the fiber 24 which transmits the laser energy along it and applies the laser energy to the area that is undergoing treatment.

From the foregoing, it will be seen that this invention is one well adapted to attain all the ends and objects hereinabove se forth together with other advantages which are obvious and which are inherent to the structure.

## Claims

1. A catheter (10) for insertion into the body to effect medical treatment, comprising:
an elongated catheter tube (12) having opposite ends (14, 16);
at least one optical fiber (24) extending through said catheter tube (12) and terminating in a tip (26) adapted to be disposed in the body when said catheter tube (12) is inserted therein, said optical fiber (24) having an input end opposite said tip (26);
ultrasonic transducer means (34) coupled to said input end of said at least one optical fiber (24) for applying ultrasonic signals to said input end of said optical fiber (24) for transmission therethrough to said tip (26) and for receiving reflected ultrasonic signals received by said tip (26) and transmitted through said optical fiber (24) to said input end, thereby providing an ultrasonic image of the treatment area adjacent said tip (26); and
means for applying laser energy (40) to said input end of said at least one optical fiber (24) to effect treatment of the area adjacent said tip (26),
**characterized in that** said means for applying laser energy (40) include a filament (42) extending through said transducer means (34) and connecting said means for applying laser energy with said optical fiber (24), said means for applying laser energy (40) being operable to apply laser energy to said filament (42).

## Patentansprüche

1. Katheter (10) zum Einführen in den Körper, um eine medizinische Behandlung durchzuführen, umfassend:
eine längliche Katheterröhre (12) mit einander gegenüberliegenden Enden (14, 16),
mindestens eine Lichtleitfaser (24), die sich durch die Katheterröhre (12) erstreckt und in einer Spitze (26) endet, darauf ausgerichtet, im Körper positioniert zu werden, wenn die Katheterröhre (12) in ihn eingeführt wird, wobei die Lichtleitfaser (24) ein Eingangsende gegenüber der Spitze (26) aufweist,
Ultraschalltransformator-Vorrichtungen (34), die gekoppelt sind an das Eingangsende der mindestens einen Lichtleitfaser (24), zum Anwenden von Ultraschallsignalen auf das Eingangsende der Lichtleitfaser (24) zur Übertragung durch diese zur Spitze (26) und zum Empfangen reflektierter Ultraschallsignale, die von der Spitze (26) empfangen und durch die Lichtleitfaser (24) zum Eingangsende übertragen wurden, wodurch ein Ultraschallbild des der Spitze (26) benachbarten Behandlungsbereichs geliefert wird, und
Vorrichtungen zum Anwenden von Laserenergie (40) auf das Eingangsende der mindestens einen Lichtleitfaser (24), um eine Behandlung des der Spitze (26) benachbarten Bereichs durchzuführen,
**dadurch gekennzeichnet, dass** die Vorrichtungen zum Anwenden von Laserenergie (40) ein Filament (42) einschließen, das sich durch die Transformator-Vorrichtungen (34) erstreckt und die Vorrichtungen zum Anwenden von Laserenergie mit der Lichtleitfaser (24) verbindet, wobei die Vorrichtungen zum Anwenden von Laserenergie (40) dafür eingerichtet sind, Laserenergie auf das Filament (42) anzulegen.

## Revendications

1. Cathéter (10) destiné à être inséré dans le corps pour réaliser un traitement médical, comprenant :
un tube de cathéter allongé (12) ayant des extrémités opposées (14, 16);
au moins une fibre optique (24) qui s'étend dans ledit tube de cathéter (12) et qui se termine par une pointe (26) conçue pour être disposée dans le corps quand ledit tube de cathéter (12) y est inséré, ladite fibre optique (24) ayant une extrémité d'entrée opposée à ladite pointe (26) ;
un moyen formant transducteur ultrasonore (34) couplé à ladite extrémité d'entrée de ladite au moins une fibre optique (24) pour appliquer des signaux ultrasonores à ladite extrémité d'entrée de ladite fibre optique (24) pour leur transmission par celle-ci à ladite pointe (26) et pour recevoir des signaux ultrasonores réfléchis reçus par ladite pointe (26) et transmis par ladite fibre optique (24) à ladite extrémité d'entrée, en produisant ainsi une image ultasonore de la zone de traitement adjacente à ladite pointe (26) ; et
un moyen pour appliquer de l'énergie laser (40) à ladite extrémité d'entrée de ladite au moins une fibre optique (24) pour réaliser un traitement de la zone adjacente à ladite pointe (26),
**caractérisé en ce que** ledit moyen pour appliquer de l'énergie laser (40) comprend un filament (42) qui s'étend au travers dudit moyen formant transducteur (34) et qui relie ledit moyen pour appliquer de l'énergie laser à ladite fibre optique (24), ledit moyen pour appliquer de l'énergie laser (40) pouvant fonctionner pour appliquer de l'énergie laser audit filament (42).
